# EUROPEAN PATENT APPLICATION

(11) **EP 2 363 111 A1**
(43) Date of publication of application: **07.09.2011**
(21) Application number: 10155083.8
(22) Date of filing: 01.03.2010
(51) Int. Cl.: A61K 9/00, A61K 31/663

(54) **Stable pharmaceutical composition comprising bisphosphonate**

(71) Applicant: Combino Pharm, S.L., 08970 Saint Joan Despi (ES)
(72) Inventor: Díaz Guasch, Laura, 08026, Barcelona (ES); Lloret Pérez, Sergio, 08030, Barcelona (ES)
(74) Representative: ZBM Patents

(57) **Abstract**

It comprises a stable liquid pharmaceutical product in a glass container, said pharmaceutical product comprising bisphosphonate, obtainable by a process comprising: (i) mixing the bisphosphonate with at least one pharmaceutically acceptable excipient in an appropriate solvent to obtain a mixture, (ii) filling the mixture into the glass container, (iii) terminally heat sterilizing the filled glass container, and (iv) cooling the sterilized filled glass container within maximum 5 hours to at least room temperature.

## Description

The present invention relates to a stable liquid pharmaceutical product in a glass container comprising bisphosphonate, and to a process for the preparation and utilization thereof.

### BACKGROUND OF THE INVENTION

Bisphosphonates are a class of compounds used to strengthen bone. Bone is in a constant state of remodelling, whereby new bone is laid down by cells called osteoblasts while old bone is removed by cells called osteoclasts. Bisphosphonates inhibit bone removal (resorption) by the osteoclasts or bone-resorbing cells. Bisphosphonates are used to prevent and treat osteoporosis and the bone pain from diseases such as metastatic breast cancer, multiple myeloma, and Paget's disease.
The bisphosphonates include zoledronate, alendronate, pamidronate, etidronate, clodronate, tiludronate, neridronate, olpadronate, ibandronate, risedronate. Customary bisphosphonate dosage forms are intravenous solutions, due to the poor absorption from the gastrointestinal system. Parenteral composition of bisphosphonate can be prepared from bisphosphonic acid or one of its salts or esters in the form of concentrate to prepare solution for infusion, ready-to use solution and others.

It is well known that bisphosphonates react with di and polyvalent cations, especially calcium, barium, magnesium, aluminium, boron and silicon. It was believed that glass may leach cations which may react with bisphosphonates and thereby inhibit its therapeutic effectiveness.

WO 2005/025551 tries to solve this aparent problem with compositions of bisphosphonates, which may be formulated for long term storage in containers comprising polymeric material and which containers would not chemically interact with bisphosphonates and which could be conveniently terminally sterilised. It is well known that the use of polymeric material for the container increases significantly the manufacturing time, production cost and furthermore cost of the final medicament. Further disadvantage is that the terminal sterilization process of polymeric material container requires either special, expensive material that allows sterilization process that fulfils Eur.Ph preferable requirements for sterile products and sterilization methods, or a long process under lower temperatures which does not fulfil Eur.Ph preferable requirements and necessitates additional validation procedures.

Another liquid formulation containing diphosphonic acid or physiologically acceptable salt or ester as the active substance is disclosed in US 5662918. More precisely US 5662918 describes injection solutions that are stable when stored in glass packaging, but where the pH of the solution is in a non-physiological grade of about 3 to 4,5, and where polyethyle glycols, which are unnecessary and not recommended in parenteral preparation pharmaceutical excipients, are used as stabilizer of the solution.

Aredia^{®}, the commercially supplied pamidronate product is a lyophilized powder that must be reconstituted with a pharmaceutically acceptable solvent before administration to a patient. Problems associated with lyophilized formulations include a risk of microbial contamination during reconstitution and an inability to terminally sterilize the drug product. Another disadvantage is the dissolving of the powder and therefore prolonged shaking may be required.

Therefore there is still a need for bisphosphonate liquid parenteral formulations, which are stable in a glass container, charactarized by having physiological pH, which are easy to prepare using only necessary pharmaceutical excipients and are easy for administration.

### SUMMARY OF THE INVENTION

In a first aspect the present invention provides a stable liquid pharmaceutical product in a glass container comprising bisphosphonate, obtainable by a process comprising: (i) mixing the bisphosphonate with at least one pharmaceutically acceptable excipient in an appropriate solvent to obtain a mixture, (ii) filling the mixture into the glass container, (iii) terminally heat sterilizing the filled glass container, and (iv) cooling the sterilized filled glass container within maximum 5 hours to at least room temperature. The pharmaceutical product can comprise a mixture of different bisphosphonates, preferably it comprises only one bisphosphonate. Further the pharmaceutical product can comprise one or more other active ingredients.

The term "stable liquid pharmaceutical product" as used throughout the specification and claims, is meant to refer to pharmaceutical product of the present invention which is chemically stable and that bisphosphonate in the product does not react with di- and polyvalent cations, and therefore no particles formation, and no turbidity occurs during shelf life of the product, thereby providing its therapeutic effectiveness and safety.

Glass containers have historically been widely used for primary pharmaceutical packaging for many reasons. Because they are transparent, pharmaceutical products can be easily visualized through the container itself. Glasses designed for pharmaceutical application are also chemically very resistant, and they are virtually impermeable to gaseous penetration. Furthermore glass can withstand very high temperatures, facilitating easy sterilization. Therefore it was very important to provide the stable liquid pharmaceutical product comprising bisphosphonate in the glass container.

The process of the invention comprises the steps of (i) mixing the bisphosphonate with at least one pharmaceutically acceptable excipient in an appropriate solvent to obtain a mixture, which is a solution or suspension or emulsion. The mixing step of the process should be carried out in an adequate container like a tank or vessel under apropriate temperature conditions and speed of the propeler if used, assuring a safe and efficient process. After mixing, the prepared mixture is filtered through adequate filters to separte undissolved particles from the mixture if necessary. Further the process of the invention comprises the step of (ii) filling the mixture into the glass container, which means transfer of the mixture into preferably previously sterilized final packaging material which is a glass container which can be of any form acceptable for pharmaceutical use. The product in its final container is then subjected to (iii) a terminal heat sterilization process followed by (iv) cooling the sterilized filled glass container comprising bisphosphonate within maximum 5 hours to at least room temperature. The term "pharmaceutical acceptable excipient" as used herein contemplates any known pharmaceutical substance for use in preparation of dosage form choosed from carriers, chelating agents, bulking agents, protectants, tonicity adjusters, buffers and pH adjusting agents, stabilizers, antimicrobial preservatives and special additives. The use of added substances falls under specific guidelines of use and are restricted in certain parenteral products.

The term "solvent" as used herein means suitable aqueous or non-aqueous vehicles and cosolvents used for dissolving or suspending or emulsifying bisphosphonate. Solvents used in the process of the present invention must meet special purity and other standards ensuring their safety by injection. At least one solvent should be selected for the process, which solvent must be nonirritating, not sensitizing and nontoxic in the amounts administered. The most frequently used solvent is water for injection as aqueous solvent. Examples of nonaqueous solvents are like vegetable oils, glycerine, alcohols and a number of less often used agents including ethyl oleate, isopropyl myristate, dimethyl acetamide.

Terminal sterilization must be a method of first choice for chemically stable pharmaceutical products. There are varieties of terminal sterilisation processes, such as for example by heat, chemical, by ionizing radiation. The commonly used ethylene oxide has been banned in the European Community as well as several countries outside of Europe because of the risk for cancer development in the human body while irradiation still meets adverse consumer-acceptance. Only the application of saturated high pressure steam with the continuous inflow sterilization process provides the ideal combination of inactivation of enzymes and the elimination of undesired micro-organisms while causing the minimum harm to the characteristics of pharmaceutical product. Steam sterilization is the preferred method of sterilization in the pharmaceutical industry. It is used for sterilization of liquid products in a wide variety of presentations. At high temperatures and in the presence of moisture the energy input from the steam inactives micro-organisms by denaturation of intracellular proteins giving certainty of sterile product. But quite frequently dosage forms cannot withstand these treatments without loss of efficacy, because of the ingredients of the composition, the manufacturing process or the packaging material. The present invention provides a stable liquid pharmaceutical product in a glass container comprising bisphosphnate that is terminal sterilized, where the terminal sterilization is heat sterilization, preferably steam sterilization. Additionally, no special or expensive equipment is necessary to carry out the heat sterilization since the glass container can withstand standard heat sterilisation conditions.

Furthermore special attention should be given to the fact that the stable liquid pharmaceutical product of invention, during manufacturing process comprises the step of cooling, which is characterized in that the cooling step is performed within maximum 5 hours. Cooling of the present invention is performed after terminal heat sterilization process and is based on decreasing the temperature of the sterilized product to at least room temperature, where room temperature is of the common range of 10°C and 38°C, preferably room temperature is considered to be 20 °C to 25 °C. It has been found that cooling the product within maximum 5 hours directly after the heat sterilization process significantly decreases reactivity between the pharmaceutical product composition and the glass container. It was also surprisingly found that the cooling process prevents glass leaching cations like calcium or silicate into solution that could react with the bisphosphonates inhibiting the therapeutic effectiveness of active compound. In the present invention the terminal heat sterilization of the product is preferably instantly followed by a cooling step which may be performed for example with cold water or sterile air in the shaking- fluid bed dryer / cooler which is directly connected to the sterilizer, or in an evaporative air cooler, or by any known system which can cool the terminally heat sterilized liquid pharmaceutical product in the glass container comprising bisphosphonate. The temperature of the product which is over 100°C after sterilization process is decreased to at least room temperature during the cooling process.

Glass can be classified based on its properties of reactivity and its composition. Type I glass is defined as a borosilicate glass with a high degree of hydrolytic stability due to its chemical composition. Type II glass and type III glass are both made of the less expensive soda-lime glass, but they are adequate for many applications and type II glass with proper treatment of the surfaces, achieves the hydrolytic stability of type I glass. It was found that type I and type II glass are particularly useful for the present invention. Preferably, the glass container of the invention is of type I or type II glass which is preferably glass ampoule, glass vial, glass bottle, glass syringe. In a preferred embodiment the stable liquid pharmaceutical product in a glass container comprising bisphosphonate is bisphosphonic acid or its salt or ester. The bisphosphonates include zoledronate, alendronate, pamidronate, etidronate, clodronate, tiludronate, neridronate, olpadronate, ibandronate, risedronate. Preferably, the stable liquid pharmaceutical product in a glass container comprises bisphosphonate, which is zoledronic acid.

The pH grade is important in liquid, drug-product formulation, especially since it involves drug solubility, activity, absorption, stability, sorption and patient comfort. The pH is also related to certain physical characteristics. The pH can be adjusted by using acidifying and alkalizing agents. Acidifying agents like for example glacial acetic acid, acetic acid, citric acid, fumaric acid, hydrochloric acid, lactic acid, malic acid, nitric acid, phosphoric acid, propionic acid, sodium phosphate, sulfuric acid, tartaric acid, are used in a formulation to lower the pH and alkalizing agents like for example ammonia solution, ammonium carbonate, diethanolamine, monoethanolamine, potassium hydroxide, sodium bicarbonate, sodium borate, sodium carbonate, sodium hydroxide, sodium phosphate dibasic, trolamine, are used to increase the pH. Many times a composition of a formulation may already have the desirable pH or may contain one of these agents to achieve a desirable pH. In all cases, before finishing up preparing a liquid formulation, the pH must be checked and adjusted if needed. Generally, because of easy incorporation, freshly prepared solutions of these agents can be used to adjust pH in such formulations. It should be noted that it is very important that pharmaceutical products comprising active compounds which are chemically stable like bisphonates, have a physiological pH range. Extremes in pH values of mixture may affect the glass container and cause formation of particles. It can also be the reason of painful administration for the patients, and vain inflammation problems. In the present invention the stable liquid pharmaceutical product comprising bisphonsphonate during the process of it's preparation comprises if needed the step of adjusting the pH of the mixture of the above step (i) of the process to a physiologically acceptable pH value of pH 5.5-8.0, preferably 6.0-7.0. This assures that product possesses sufficient buffer capacity to maintain the pH of physiological value in order to ensure a high quality of the product based on characteristics that define safety and efficacy.

In a preferred embodiment of the invention, the cooling step of the process of the invention is performed within 3 hours, more preferably in 1 hour which assures no reactivity between the mixture and the glass container and also avoids problems of unacceptable particulate formation, turbidity.

The stable pharmaceutical product that comprises bisphosphonic acid or its salt or ester can be in the form of solution, suspension, dispersion, emulsion or in any pharmaceutical dosage form for parenteral use which is a liquid form. But in preferred embodiment, the stable liquid pharmaceutical product in a glass container comprising bisphosphonate is in the form of a solution. Precisely the stable pharmaceutical product of the invention is preferably in the form of a solution for parenteral administration. Further the solution is preferably in the form of concentrate for intravenous infusion or a ready to use solution for intravenous infusion providing the product of highest quality and avoiding the need for the practitioner to reconstitute the active agent at the time of administration.

Throughout the description and claims the word "comprise" and variations of the word, are not intended to exclude other technical features, additives, components, or steps.

Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

### Example 1.

Preparation of the pharmaceutical product in a glass container comprising a concentrate for the solution for infusion of zoledronic acid.

**Table. 01. Pharmaceutical composition of Batch 01 and Batch 02.**

| Composition | [mg/vial 5ml] |
|---|---|
| Zoledronic acid | 4 mg |
| Mannitol injectable | 220 mg |
| Sodium citrate dihydrate | 24 mg |
| Water for injection | up to 5 ml |
| Magnesium stearate | 1.5 mg |

| | |
|---|---|
| * 4 mg of anhydrous Zoledronic acid equivalent to 4,26 mg of Zoledronic acid monohydrate. | |

The concentrate for the solution for infusion of zoledronic acid was manufactured using the following procedure comprising the following steps: i) dissolving in 80% of total volume of water for injection, mannitol, zoledronic acid and sodium citrate, pH of the solution was controlled maintaining physiological grade 6.0-7.0, adding the rest of water for injection, (ii) sterile filtration of the solution through PVDF 0,45 µm and 0,22 µm filters, filing the solution into type I 5 ml glass vials and closing the glass vials with butyl bromide rubber stoppers and aluminium seals, (iii) terminal steam sterilization in autoclave at 121°C during 20 minutes (iv) cooling within maximum 5 hours to at least room temperature, of the sterilized product, performed in autoclave using cold water. Cooling process conditions were as follows: temperature of water: 1°C, process duration: 1 hour, sterilized product initial temperature 121°C, sterilized product terminal temperature 25 °C.

### Example 2.

### Stability results of the pharmaceutical product in glass container comprising zoledronic acid.

Batch 01 of the pharmaceutical product in the glass container comprising zoledronic acid produced according to example 1. was subjected to accelerated and long term stability tests. The stability studies were carried out with the following conditions as is define in ICH Topic Q1A "Stability Testing of New Drug Substances and Products" (CPMP/ICH/2736/99).
25 ± 2°C & 60% ± 5% relative humidity ― Condition A.
40 ± 2°C & 75% ± 5% relative humidity ― Condition B.

**Table 2. Stability results of Batch 01**

| **Time (months)** | | **0** | **3** | | **6** | | **9** | **12** |
|---|---|---|---|---|---|---|---|---|
| **Condition** | | | **A** | **B** | **A** | **B** | **A** | **A** |
| **Test** | **Requirement** | | | | | | | |
| Appearance | *Clear solution free of particles* | Compl. | Compl. | Compl. | Compl. | Compl. | Compl. | Compl. |
| Colour of solution | *Not more than Y₆* | Compl. | Compl. | Compl. | Compl. | Compl. | Compl. | Compl. |
| pH | *Between 6.0 and* 7.0 | 6.2 | 6.3 | 6.3 | 6.3 | 6.3 | 6.3 | 6.3 |
| Uniformity of Dosage | *Average 10* ≤ *15* | 2.4 | 2.4 | 2.1 | 1.0 | 2.1 | 1.0 | 0.7 |
| | *No individual value out of (1* ± *25x 0.01)M* | | | | | | | |
| Assay (%) | *Between 95. 0-105.0% of labelled amount* | 101.4 | 102.4 | 102.2 | 100.4 | 99.0 | 98.1 | 100.2 |
| Related substances | | | | | | | | |
| Imidazole | *Not more than 1.0%* | ND | ND | ND | ND | ND | ND | ND |
| 1-Imidazole | *Not more than 1.0%* | ND | ND | ND | ND | ND | ND | ND |
| Individual impurities | *Not more than 0.5%* | ND | ND | ND | ND | ND | ND | ND |
| Total impurities | *Not more than 3.0%* | ND | ND | ND | ND | ND | ND | ND |
| Extractable Volume | Not less than 5 ml | > 5ml | N/A | N/A | > 5ml | > 5ml | N/A | > 5ml |
| Turbidimetry | *Not more than 3 NTU* | 1.14 | N/A | N/A | N/A | 0.54 | N/A | 0.62 |
| Determination of Ca²⁺ | *Not more than 8.5 ppm* | 0.29 | N/A | N/A | N/A | 0.68 | N/A | 0.41 |
| Endotoxins | *Not more than 70.0 EU*/*ml* | <70 EU/ml | N/A | N/A | N/A | <70 EU/ml | N/A | <70 EU/ml |
| Sterility | *Sterile* | Sterile | N/A | N/A | N/A | Sterile | N/A | Sterile |
| Particles | *Not less than 25 µm-Not more than 600 part per vial* | 1 | N/A | N/A | N/A | 12 | N/A | 45 |
| | | | | | | | | |
| | *Not less than 10 µm-Not more than 6000 part per vial* | 147 | | | | 206 | | 275 |

| | | | | | | | | |
|---|---|---|---|---|---|---|---|---|
| N/A- not applicable, ND- not detected. Compl.-complies | | | | | | | | |

All the tests were performed according to methods based on Eur.Ph. references and ICH guidelines requirements. It can be seen that stability results of all tested parameters comply the requirements. It should be noted that critical parameters like particles, cations leaching into solution (determination of Ca²⁺ content in the solution), turbidimetry are well below the specified limits ensuring high quality of the product comprising bisphosphonate in the glass container.

### Example 3.

### Stability results of the pharmaceutical product in glass container comprising zoledronic acid.

Batch 02 of the pharmaceutical product in the glass container comprising zoledronic acid produced according to example 1. was subjected to accelerated and long term stability tests. The stability studies were carried out with the following conditions as is define in ICH Topic Q1A "Stability Testing of New Drug Substances and Products" (CPMP/ICH/2736/99).
25 ± 2°C & 60% ± 5% relative humidity ― Condition A.
40 ± 2°C & 75% ± 5% relative humidity ― Condition B.

**Table 3. Stability results of Batch 02**

| **Time (months)** | | **0** | **3** | | **6** | |
|---|---|---|---|---|---|---|
| **Condition** | | | **A** | **B** | **A** | **B** |
| **Test** | **Requirement** | | | | | |
| Appearance | *Clear solution free of particles* | Complies | Complies | Complies | Complies | Complies |
| Colour of solution | *Not more than Y₆* | Complies | Complies | Complies | Complies | Complies |
| pH | *Between 6.0 and 7.0* | 6.2 | 6.3 | 6.3 | 6.3 | 6.3 |
| Uniformity of Dosage | *Average 10* ≤*15* | | | | | |
| | | | | | | |
| | *No individual value out of (1*±*25x0.01)* M | 0.8 | 3.1 | 9.4 | 2.6 | 3.3 |
| Assay (%) | *Between 95.0-105.0% of labelled amount* | 99.3 | 99.3 | 101.1 | 99.4 | 98.3 |
| Related substances | | | | | | |
| Imidazole | *Not more than 1.0%* | ND | ND | ND | ND | ND |
| 1-Imidazole | *Not more than 1.0%* | ND | ND | ND | ND | ND |
| Individual impurities | *Not more than 0.5%* | ND | ND | ND | ND | ND |
| Total impurities | *Not more than 3.0%* | ND | ND | ND | ND | ND |
| Extractable Volume | Not less than 5 ml | >5 | N/A | N/A | N/A | >5 |
| Turbidimetry | *Not more than 3 NTU* | 0.28 | N/A | N/A | N/A | 0.30 |
| Determination of Ca²⁺ | *Not more than 8.5 ppm* | 0.12 | N/A | N/A | N/A | 0.31 |
| Endotoxins | *Not more than 70.0 EUlml* | <70 EU/ml | N/A | N/A | N/A | <70 EU/ml |
| Sterility | *Sterile* | Sterile | N/A | N/A | N/A | Sterile |
| Particles | *Not less than 25 pm-Not more than 600 part per vial* | 6 | N/A | N/A | N/A | 1 |
| | *Not less than 10 µm-Not more than 6000 part per vial* | 415 | | | | 35 |

| | | | | | | |
|---|---|---|---|---|---|---|
| N/A- not applicable, ND- not detected. | | | | | | |

All the tests were performed according to methods based on Eur.Ph. references and ICH guidelines requirements. It can be seen that stability results of all tested parameters comply the requirements and no precipitation were observed. It should be noted that critical parameters like particles formation, turbidimetry, cations leaching into solution (determination of Ca²⁺ content in the solution), are well below the specified limits, ensuring high quiality of the product comprising bisphosphonate in the glass container.

## Claims

1. A stable liquid pharmaceutical product in a glass container, said pharmaceutical product comprising bisphosphonate, obtainable by a process comprising: (i) mixing the bisphosphonate with at least one pharmaceutically acceptable excipient in an appropriate solvent to obtain a mixture, (ii) filling the mixture into the glass container, (iii) terminally heat sterilizing the filled glass container, and (iv) cooling the sterilized filled glass container within maximum 5 hours to at least room temperature.

2. The stable liquid pharmaceutical product according to claim 1, **characterized in that** the glass container is of type I or type II glass.

3. The stable liquid pharmaceutical product according to any of the claims 1-2, **characterized in that** the glass container is a glass ampoule, glass vial, glass bottle or a glass syringe

4. The stable liquid pharmaceutical product according to any of the claims 1-3 **characterized in that** the bisphosphonate is bisphosphonic acid or its salt or ester, preferably zoledronic acid.

5. The stable liquid pharmaceutical product according to any of the claims 1-4, **characterized in that** the process additionally comprises the step of adjusting the pH of the mixture of step (i) to a physiologically acceptable pH value of pH 5.5-8.0, preferably 6.0-7.0.

6. The stable liquid pharmaceutical product according to any of the claims 1-6, **characterized in that** the fast cooling step of the process is performed within 3 hours, more preferably in 1 hour.

7. The stable liquid pharmaceutical product according to any of the claims 1-6, **characterized in that** the mixture is in the form of a solution, suspension, emulsion.

8. The stable liquid pharmaceutical product according to claim 7, **characterized in that** said product is for parenteral administration.

9. The stable liquid pharmaceutical product according to claim 8, **characterized in that** the said product is in the form of concentrate for intravenous infusion or a ready to use solution for intravenous infusion.
